(19) Europäisches Patentamt
European Patent Office
Office européen des brevets

(11) Publication number: 0 512 404 A1

# (12) EUROPEAN PATENT APPLICATION

(21) Application number: 92107346.6

(22) Date of filing: 29.04.92

(51) Int. Cl.5: C10L 1/00, C10M 171/00, C10L 1/22, C10L 1/24, G01N 30/70

(30) Priority: 03.05.91 US 695576

(43) Date of publication of application: 11.11.92 Bulletin 92/46

(84) Designated Contracting States: AT BE DE ES FR GB IT NL

(71) Applicant: NALCO CHEMICAL COMPANY
One Nalco Center
Naperville Illinois 60563-1198(US)

(72) Inventor: Brinkman, Kerry C.
8907 Tavistock Dr.
Houston, Texas 77031(US)
Inventor: Enderson, Dayle M.
1406 Ravenscourt Dr.
Sugar Land, Texas 77478(US)

(74) Representative: Ruschke, Olaf et al
Patentanwälte Dipl. Ing. Olaf Ruschke Dipl. Ing. Hans E. Ruschke Dipl.-Ing. Jürgen Rost Dipl.-Chem Dr. U. Rotter Pienzenauerstrasse 2
W-8000 München 80(DE)

## (54) Identification of liquid hydrocarbons using chemical markers.

(57) The present invention relates to a composition, a method for preparing a composition, and a method for analyzing the composition where the composition includes a liquid hydrocarbon and one or more marker compounds of formula (I):

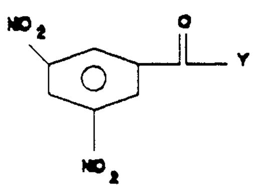

where Y is selected from X-$R^2$ or $NR^3R^4$, and where X is an oxygen atom or a sulfur atom, $R^2$ is an organic radical having from about 1 to about 30 carbon atoms, $R^3$ and $R^4$ are the same or different organic radical having from about 1 to about 30 carbon atoms. The markers can be present in the amounts from about .05 parts per million to about 50 parts per million, preferably from about .05 to about 25 parts per million and preferably from about 1 to about 10 parts per million and is usable to identify not only the source and identity of the particular hydrocarbon liquid, but also to allow expedient tracing of the hydrocarbon back to its source and analysis of the particular types of additives that may be present and to what extent they are present in the hydrocarbon.



Rank Xerox (UK) Business Services

## FIELD OF THE INVENTION

This invention relates to the use of specific chemical compounds as chemical markers to be added to liquid hydrocarbons in low concentrations in order to uniquely mark the liquid hydrocarbon so that it can be quickly identified with respect to its source and integrity. The chemical markers can also be premixed with any performance enhancement package to further allow identification of the type and amount of the package present in the hydrocarbon liquid.

## BACKGROUND OF THE INVENTION

Numerous techniques have been used for marking liquid hydrocarbons for the purpose of identifying its source and integrity. These methods typically involve the incorporation of small amounts of chemical additives or markers to the liquid. Subsequent detection of the markers can be achieved by a number of standard analytical techniques such as colorimetry, IR, UV or UV-Vis spectroscopy, mass spectrometry, neutron activation or atomic adsorption spectroscopy. Radioactive tracers or markers have also been used including the addition of tritium, iodine-131, or sulfur-35, in order to trace fuels in pipelines and in oil storage facilities.

There are various circumstances when it would be advantageous to determine the presence and concentration of a substance which had previously been added to a hydrocarbon liquid. The presence and concentration of performance-enhancing additives in a hydrocarbon might be inferred by measuring the concentration of a specific substance that was co-injected into the hydrocarbon along with the primary additive. Also, if a hydrocarbon liquid were accidentally discharged into the environment, the source of the hydrocarbon might be determined by identifying a specific unique substance that had previously been added to the hydrocarbon. Similarly, the uniquely marked hydrocarbon could be monitored throughout its distribution network to verify its identity and aid in quality assurance programs that seek to prevent contamination with other hydrocarbon liquids. For example, motor oil consists of a major amount of paraffinic hydrocarbon and a minor amount of a mixture of performance-enhancing additives. However, once mixed, it is difficult to determine the concentration of the additive package in the oil. If a known amount of an easily detectable substance were included in the additive package, the concentration of the package in the oil could be inferred by determining the quantity of marker substance in the finished motor oil. This would simplify quality control measurements and allows identification of the oil once in service. This can also be applied to additives that are blended into gasoline, diesel fuels and other hydrocarbons.

In another example, environmental damage occurs when crude oil washes up on shore as a result of accidental or intentional discharges from tanker vessels. If all tanker vessels were required to inject small amounts of a marker substance into the hydrocarbon when on-loading their cargo, the source and responsibility of all spills could be determined and appropriate action taken.

Environmental damage and poisoning of ground water can result from the leakage of gasoline or fuel oils from storage tanks. To determine the extent and location of such leakage, it would be desirable if the soil, fluid or ground water adjacent to the storage vessel could be collected and then analyzed to determine if the fluid was escaping from the vessel. Such leaks commonly occur in tank farms, service station underground tanks, pipelines and the like.

In U.S. Patent No. 4,141,692, Keller described the use of chlorinated hydrocarbon marking agents in liquid hydrocarbon environments. The marking agents were restricted to chlorinated hydrocarbons having at least 3 chlorine atoms, at least two carbon atoms and a Cl/C atom ratio of at least 1 to 3. The components were detectable using a electron capture detector after gas chromatographic separation. Although the compounds were described as relatively non-toxic, the use of chlorinated hydrocarbons as markers has certain disadvantages. The most obvious disadvantage is that chlorinated markers used in liquid hydrocarbon fuels can produce potentially toxic oxidative break down products when the fuel is burned. The break down products also will generally add to increased amounts of chlorinated hydrocarbon emissions into the atmosphere, a potential risk to the ozone layer.

In U.S. Patent No. 4,209,302, Orelup described the use of 1-(4-morpholino)-3-(α or β-naphthylamino) propanes in the concentration range from 0.5 to 12 part per million (ppm) as potential marking agents for gasoline. These markers did not impart a color to the gasoline, but required "wet chemical" extraction followed by subsequent treatment with diazotized 2-chloro-4-nitroaniline to yield a pink solution which could be measured colorimetrically. The major disadvantage of this technique is the necessity for performing a preliminary chemical extraction prior to identification and quantitation which can lead to increased errors in measurement.

SUMMARY OF THE INVENTION

This invention relates to a liquid composition containing a liquid hydrocarbon and one or more marker compounds or agents of formula (I) below:

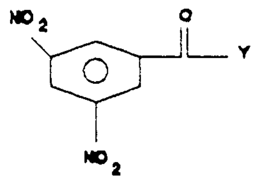

where Y is selected from X-$R^2$ or $NR^3R^4$, and where X is an oxygen atom or a sulfur atom, $R^2$ is an organic radical having from about 1 to about 30 carbon atoms, $R^3$ and $R^4$ are the same or different organic radical having from about 1 to about 30 carbon atoms.

The present invention also relates to a method for preparing and a method for analyzing the composition with respect to the marker compound(s) identity and quantity. Optionally, the liquid hydrocarbon can contain a performance enhancing package and the marker(s) can be added to the package prior to introducing the package into the hydrocarbon liquid.

The marker is added in sufficient quantities to ensure that identification and quantitation of the marker can be achieved. Typically, the marker is added in a range from about 0.5 to about 50 parts per million (ppm) based on the weight of liquid hydrocarbon to be marked. The addition of the chemical marker(s) allows the source and integrity of a hydrocarbon liquid to be determined as well as indirectly the type and/or amount of any performance enhancement material or package added to a particular liquid hydrocarbon to be determined.

Thus, an appropriately marked hydrocarbon liquid can be analyzed to guard against mixing it with different types of liquids. The hydrocarbon liquid can include, but is not limited to, gasoline, lubricating oils, crude oil, and the like. The markers can also be used to identify the source of a liquid hydrocarbon which has been previously marked by a marking agent of the present invention. The source identification can be used to assess responsibility and potential liability for environmental damages. Analysis of the marked liquid can also be used to determine the type and nature of any liquid hydrocarbon leaks or spills.

DESCRIPTION OF THE PREFERRED EMBODIMENT

The composition of the present invention comprises a liquid hydrocarbon and one or more marker compound or agent, said marker being present in sufficient quantity to facilitate subsequent analytical identification and quantitation and typically ranging from about 0.5 ppm to about 50 ppm based on the weight of the liquid hydrocarbon. Preferably, the markers of the present invention are present in amount from about 0.5 ppm to about 25 ppm and more particularly, from about 1 ppm to about 10 ppm. However, lesser and greater amount can be used. The limitation on the low end regarding quantity is determined by the sensitivity of the analytical detectors. The higher amount is controlled by convenience. The markers are required to be soluble in the liquid hydrocarbon at the particular levels chosen in order that accurate measurements can be made.

The method of preparing a composition of the present invention comprises the steps of adding one or more markers to a liquid hydrocarbon under mixing condition sufficient to facilitate admixing of the mixture into said liquid, said marker being present in sufficient quantity to facilitate subsequent analytical identification and quantitation typically ranging from about 0.5 ppm to about 50 ppm based on the weight of the liquid hydrocarbon to be marked. Preferably, the markers of the present invention are present in amount from about 0.5 ppm to about 25 ppm and more particularly, from about 1 ppm to about 10 ppm. The hydrocarbon liquid can optionally contain a performance enhancing package and the markers can be added to the package prior to adding the package to the liquid.

The method for analyzing the liquid composition of the present invention comprises the steps of analyzing a sample of said liquid composition by an analytical method, said liquid composition comprising a liquid hydrocarbon and one or more markers in sufficient quantity to facilitate subsequent analytical identification and quantitation and said analytical method being sufficient to determine the identity and concentration of the marker(s) contained in the liquid composition. The markers can be present in the range

from about 0.5 ppm to about 50 ppm based on the weight of the liquid hydrocarbon to be marked. Preferably, the markers of the present invention are present in amount from about 0.5 ppm to about 25 ppm and more particularly, from about 1 ppm to about 10 ppm. The hydrocarbon liquid can optionally contain a performance enhancing package and the markers can be added to the package prior to adding the package to the liquid.

The analytical methods useful in the present invention include, but is not limited to, gas chromatographic separation followed by component detection using a detector such as an electron capture detector, a mass spectrometer, a flame photometric detector, electrolytic conductivity detector, microcoulometer, chemiluminescence detector, a nitrogen phosphorous detector, a photoionization detector, infra-red spectrometer, ultra violet spectrometer, fluorescence detector, microwave inductive plasma detector, gas density balance detector, ultrasonic detector or other similar detectors, high performance liquid chromatographic separation followed by component detection using one of the above listed detectors, or other analytical methods capable of identifying and quantifying the markers. Gas chromatography followed by component detection using an electron capture detector is preferred.

The markers are compounds of formula (I):

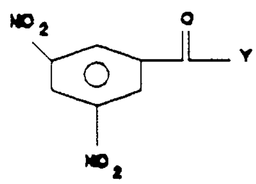

where Y is selected from X-$R^1$ or $NR^2R^3$, and where X is an oxygen atom or a sulfur atom, $R^1$ is an organic radical having from about 1 to about 30 carbon atoms, $R^2$ and $R^3$ are the same or different organic radical having from about 1 to about 30 carbon atoms. The term organic radical used to describe $R^{1-3}$ includes substituents selected from the illustrative and representative group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine. Preferably, $R^1$ is selected from the illustrative and representative group consisting of a linear or branched alkyl group having from about 1 to about 30 carbon atoms, an alkylated glycol and an alkylated polyglycol.

The liquid hydrocarbon can be selected from the illustrative and representative group consisting of crude oil, residual oil, lubricating oil, hydraulic and circulating oils, heating oil, diesel fuel and mid-distillates, jet fuel, aviation fuel, gasoline, gasohol or alcohol oxygenated gasoline components such as MTBE, fuels, polymer lattices and solutions, and bulk liquid polymers and mixtures thereof and similar hydrocarbon liquids or liquid mixtures.

The performance enhancing package can comprise mixtures of components selected from the illustrative and representative groups consisting of detergents, dispersants, corrosion inhibitors, anti-oxidants and stabilizers, wax crystal modifiers, EP and anti-wear agents, emulsion breakers and dehazers and octane enhances.

Each marker represented by formula (I) can be used independently or in combination to mark a particular liquid hydrocarbon. Thus, the methyl and ethyl derivatives could be added together or used independently as a marking agent, along with any of the other higher alkyl analogs. Alternately, a set of n markers of formula (I) can be used to establish a tagging code by adding to each distinct hydrocarbon liquid in a given sequence of quantities of each of the n markers. Thus, using 3 distinct markers of formula (I), A, B, and C, and 2 distinct quantitative values of the markers such as 1 and 2, one could uniquely tag 8 liquid hydrocarbons as shown below:

1. (1A,1B,1C); 2. (1A,1B,2C); 3. (1A,2B,1C); 4. (2A,1B,1C); 5. (1A,2B,2C); 6. (2A,1B,2C); 7. (2A,2B,1C); and 8. (2A,2B,2C).

It can readily be seen that this coding process obeys the formula of $M^n$ coding sequences where M are the allowed quantities such as 10ppm, 20ppm, 30ppm, etc. and n is the number of markers. The numeric value of n which corresponds to the number of markers used in a give sequence can range from about 2 to about 20 markers or more. The number of allowable marker quantities or levels, M, used in a given sequence can range from about 2 to about 10 different quantities or more. The exact number of markers, n,

and the number of levels, M, used in a given sequence will be dictated by convenience and simplicity. The quantitative values can be separated by an increment of from about 1 to about 20 ppm and preferably from about 5 to about 10 ppm again with necessity and convenience dictating the exact increment. Although the general formula for enumerating the coding sequences is $M^n$, when one of the allowed quantitative levels is zero, or the absence of a marker, then the number of usable sequences is reduced by one which would correspond to a sequence where no markers are added.

Compounds of Formula (I) can typically be prepared by the reaction of 3,5-dinitro benzoic acid or its reactive analogs, including the acid chloride, bromide or the anhydride, with either an alcohol, a thiol or an amine.

Thus, when Y is equal to X-$R^1$ the compound of Formula (I) can be prepared by reacting 3,5-dinitro benzoic acid or a reactive analog with an alcohol or thiol where $R^1$ is selected from the illustrative and representative group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine. Preferably, $R^1$ is selected from the illustrative and representative group consisting of a linear or branched alkyl group having from about 1 to about 30 carbon atoms, an alkylated glycol and an alkylated polyglycol.

The alcohols useful in the preparation of markers of formula (I) include, but are not limited to, methanol, 2-methoxyethanol, 2-(2-methoxyethoxy)ethanol, ethanol, 2-ethoxyethanol, 2-(2-ethoxyethoxy)ethanol, propanol, 2-propoxyethanol, 2-(2-propoxyethoxy)ethanol, 2-propanol, 2-methyl-1-propanol, 2-methyl-2-propanol, butanol, 2-butoxyethanol, 2-(2-butoxyethoxy)ethanol, 2-butanol, 2-methyl-1-butanol, 3-methyl-1-butanol, 2-ethyl-1-butanol, n-pentanol, 2-pentanol, 2-pentoxyethanol, 2-(2-pentoxyethoxy)ethanol, 2-methyl-1-pentanol, 4-methyl-2-pentanol, 2,4-dimethyl-3-pentanol, 3-methyl-3-pentanol, hexanol, 2-hexoxyethanol, 2-(2-hexoxyethoxy)ethanol, 2-hexanol, 3-hexanol, 2-ethyl-1-hexanol, 3,5,5-trimethyl-1-hexanol, heptanol, 2-heptanol, 3-heptanol, 2,6-dimethyl-4-heptanol, octanol, 2-octanol, 3-octanol, 3,7-dimethyl-1-octanol, nonanol, and decanol. The analogous thiol reagents can be used to prepare sulfur analogs of the benzoate compounds derived from the above listed alcohols.

When Y is equal to $NR^2R^3$ the compound can be prepared by reacting 3,5-dinitro benzoic acid or a reactive analog with an appropriate amine where $R^2$ and $R^3$ are the same or different and are selected from the illustrative and representative group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine. Preferably, $R^2$ and $R^3$ are selected from the illustrative and representative group consisting of a linear or branched alkyl group having from about 1 to about 30 carbon atoms, an alkylated glycol and an alkylated polyglycol.

The amines useful in the preparation of markers of formula (I) include, but are not limited to, linear primary amines such as methylamine, ethylamine, propylamine, butylamine, pentylamine, hexylamine, heptylamine, octylamine, nonylamine, decyl amine, branched primary analogs, dialkylamine such as dimethylamine, diisopropylamine, dipropylamine, dibutylamine, di-sec-butylamine, dipentylamine, dihexylamine, di-(2-ethylhexyl)amine and dioctylamine, and mixed dialkyl amines such as methylethylamine, methylbutylamine, ethylhexylamine and the like.

As mentioned previously, the preferred analytical detection method is gas chromatography (GC) using an electron capture detector (ECD) to determine the concentration and identity of the specific marker(s) in the liquid composition. The ECD detects a chemical compound by the compounds ability to capture electrons present in the detector as the compound passes out of a GC separation column and into and through the ECD.

Gas chromatography separates chemicals according to their interaction with a given GC column stationary phase at a given constant temperature or according to a given temperature program. A temperature program is a computer controlled sequence of temperature increases or decreases utilized to enhance chromatographic separation and the short analysis time. Each chemical compound interacts differently with a given stationary phase under given temperature condition which causes each particular compound to have a unique retention time, the difference between the time the composition is introduced onto the column and the time a given component exits from the column and is detected by a detector. The retention time, once determined, can be used to identify the marker while the peak area can be used to determine the concentration of the marker. The identity of the particular markers can then be used to identify the source of the particular hydrocarbon, as well as identifying the nature of the hydrocarbon liquid.

Thus, if the hydrocarbon liquid contains a performance enhancement material or package to which a marker has been added, then the concentration of the package as well as the nature of the particular package and liquid can be determined by the GC method of analysis.

The stationary phases useful to separate and identify the markers of the present invention are silicones selected from the illustrative and representative group consisting of dialkylpolysiloxanes, alkylarylpolysiloxanes, substituted alkylarylpolysiloxanes, copolymers thereof and mixtures thereof and carbowaxes, where the alkyls have from about 1 to about 10 carbon atoms, the aryls have from about 6 to about 14 carbon atoms, and the substituted alkyls are cyanoalkyls, dialkylaminoalkyls, alkoxyalkyls, and haloalkyls and where copolymers means any polymer comprising two or more of the above listed homopolysiloxanes units. Preferably, the materials are selected from the group consisting of dimethylpolysiloxane, phenylmethyl polysiloxane and cyanopropylphenyl-methyl polysiloxane.

The GC columns can be packed or unpacked both of which are standardly known in the art. The column can be coated or packed with the stationary phase as is standardly known in the art. For a general discussion of GC separation and analysis, see "Modern Practice of Gas Chromatography", edited by Robert L. Grob, published by John Wiley and Sons.

The electron capture detector is described in many publications such as the textbook entitled, "Modern Practice of Gas Chromatography", edited by Robert L. Grob, published by John Wiley and Sons (see particularly pages 255-267). Since electron capture detection is a well-known technique, it is not described here in any greater detail.

The preferred GC process of the present invention comprises the steps of:

1) injecting a liquid composition comprising a liquid hydrocarbon and one or more markers onto a first end of a first GC column through an injector port equipped with a carrier gas supply which supplies a carrier gas;

2) separating said markers from a major portion of said liquid into one or more discrete peaks on said first column;

3) opening a valve connected to a second end of said first column for one or more time windows;

4) collecting each of said markers corresponding to each of said windows into a cold trap comprising a first end of a second GC cooled by a coolant;

5) removing said coolant from said cold trap;

6) further separating said makers on said second column; and

7) detecting each of said makers with an electron capture detector equipped with a make up gas,

said first column containing a first column stationary phase and being of a first length sufficient to affect an initial or partial separation of the marker from said major portion of said liquid, said windows corresponding to one or more retention times of said markers and being of sufficient duration to allow all or nearly all, but preferably all, of each marker to pass therethrough, and said second column containing a second column stationary phase being the same or different from said first stationary phase and being of a second length sufficient to separate each of said markers from any other components in said liquid.

The valve opening is typically controlled by a computer system where the operator would input into the computer the retention times corresponding to each marker and the duration of the window during which time the valve will be in an open condition. At all other times, the valve will remain in a closed condition and the non-marker components of the liquid will either simply exit the first column or be introduced into an optional second detector usually a flame ionization detector. The duration of the window is set so that the valve opens sufficiently before the marker peak arrives at the valve and remains open sufficiently after the marker peak to insure that all or nearly all, but preferably all, of the marker peak is deposited into the cold trap.

The major portion of the liquid composition is comprised of non-polar hydrocarbons, but the composition can also contain minor polar or other components. It should be recognized that the minor polar component of the liquid could possibly be contained within any one of said windows. This possibility is one of the reasons for employing a double column GC method. The second column insures complete separation of said markers from any other components in said liquid. The second column also insures superior identification and quantitation of said markers.

The term peak as used in the present invention is meant to refer to the band or front of molecules moving through the columns that are composed primarily of the marker molecules.

The coolants useful in the present invention can be selected from the illustrative and representative group consisting of carbon dioxide, nitrogen, a freon, an alcohol, a glycol or any other similar cooling gas or liquid.

The column stationary phases are as previously described, preferentially the stationary phases are different; one phase being of a different polarity than the other phase. Thus, the first stationary phase could

be an arylalkyl polysiloxane while the second stationary phase could be a dialkyl polysiloxane or vis-a-versa. The polarity of different typically used silicones is generally known in the art. The stationary phase can either be packed into the column absorbed on an inert carrier or preferably coated on the columns to a thickness from about 0.1 to 1.5 microns.

The columns preferred for this invention are columns having a diameter from about 0.1 to 0.7 millimeters, preferably from about 0.18 to 0.53 millimeters with 0.25 to 0.53 millimeters being particularly preferred. The columns are known as capillary columns in the art. The length of the first column is preferably shorter than the length of the second column and being from about 5 to about 60 meters with from about 10 to 20 meters being preferred and from about 12 to about 18 meters being particularly preferred. The length of the second column is from about 10 to about 60 meters with 20 to 40 meters being preferred and from about 25 to about 35 meters being particularly preferred.

The injector port is maintained at a temperature sufficient to insure that all the components are vaporized before entering the first column, typically from about 250°C to about 300°C. The method can either be carried out in an isothermal condition, a constant temperature condition where the isothermal temperature can generally be from about 175°C to about 275°C. The method can also be performed using temperature programming. Temperature programming allows the temperature of the method to be either increased or decreased at a given rate measured in °C/minute. The exact temperature program used is highly subjective and any appropriate temperature programming sequence generally used in the art can be applied to the present method. Preferably, the method is performed using temperature programming.

The carrier gas is an inert gas used in GC to establish a so called mobil phase while the silicone stationary phase establishes the so called stationary phase, both of which are well known in the art and are responsible for accomplishing separation because different molecules spend different amount of time in each phase. 田 preferred carrier gas is helium and the preferred make up gas is nitrogen.

The makeup gas used by the electron capture detector is nitrogen or a mixture of argon/methane if the carrier gas is helium, nitrogen if the carrier gas is nitrogen, and nitrogen or a mixture of argon/methane if the carrier gas is hydrogen.

In order to be a useful marker for the present invention, the compound must be chlorine free and contain groups which have high electron affinities illustrated by nitro and nitrate compounds, carbonyls, nitriles and organo-metallic compounds, compounds containing conjugated double bonds, certain aldehydes and ketonic groupings having a conjugated system. A list of typical electrophoric compounds that would be illustrative of groupings and compounds capable of electron capture detection is given in "Modern Practice of Gas Chromatography" on page 266. The disclosure of which herein is incorporated by reference.

The dosage of the marker compounds containing the electrophoric groupings is within the range of 0.5-50 parts per million (ppm), with a typical dosage being 1-10 ppm. In most cases when the treated liquid hydrocarbons or the fuel into which the additive package has been placed are tested, it is possible to detect the presence of the marker compound at a dosage as low as 0.5 ppm. In certain instances even lower dosages can be detected.

The present invention can be further understood and additional aspects can be discerned from the following illustrative and representative examples.

PREPARATION OF MARKERS OF FORMULA (I)

EXAMPLE 1

This example illustrated the general preparation of 3,5-dinitrobenzoates and thio benzoates by esterification of 3,5-dinitrobenzoic acid with a suitable alcohol or thiol. This particular example depicts the preparation of 2-ethylhexyl-3,5-dinitrobenzoate.

To a 250 Ml 4-necked glass reaction flask with a stirring paddle, thermometer, dean stark trap with condenser and a gas inlet adaptor was added approximately 106.1 grams (0.5 moles) of 3,5-dinitrobenzoic acid, 71.6 grams of 2-ethylhexanol (0.55 moles) and 0.1 grams of methane sulfonic acid. The mixture was heated to 140°C and mechanically stirred. After one hour, vacuum was slowly applied to the vessel until a mild reflux was obtained. The reflux was continued for two hours at 140°C and water was collected in the dean stark trap until the acid value of the pot material was below 8 milligrams potassium hydroxide (KOH) per gram of material (95% esterification). The vessel was cooled below 100°C and then 50 Ml of 1.0 M aqueous potassium carbonate was added. After thorough mixing, the base wash was discarded. After a subsequent water wash, the vessel was reheated to 140°C and vacuum was applied to remove any residual water and alcohol.

EXAMPLE 2

This example illustrated the preparation of n-decyl-3,5-dinitrobenzoates according to the procedure described in Example 1 except 10.60 grams (0.05 moles) of 3,5-dinitrobenzoic acid and 8.72 grams (0.055 moles) of decyl alcohol were used.

EXAMPLE 3

This example illustrated the preparation of the 2-(2-hexoxyethoxy)ethyl-3,5-dinitrobenzoate according to the procedure described in Example 1 except 10.60 grams (0.05 moles) of 3,5-dinitrobenzoic acid and 9.6 grams (0.055 moles) of 2-(2-hexoxyethoxy)ethanol were used. This reaction can also be performed by using a heterogeneous acidic catalyst instead of methyl sulfonic acid such as Amberlyst 15.

EXAMPLE 4

This example illustrated the preparation of the 2-hexoxyethyl-3,5-dinitrobenzoate according to the procedure described in Example 1 except 42.42 grams (0.2 moles) of 3,5-dinitrobenzoic acid, 30.71 grams (0.21 moles) of 2-hexoxyethanol, 5 grams of Amberlyst 15 and 25 Ml of toluene were used.

EXAMPLE 5

This example illustrated the preparation of 2-(2-ethoxyethoxy) ethyl-3,5-dinitrobenzoates according to the procedure described in Example 1 except 42.42 grams (0.2 moles) of 3,5-dinitrobenzoic acid, 28.28 grams (0.21 moles) of 2-(2-ethoxyethoxy)ethanol, 5 grams of Filtol 13 LM (as catalyst) and 25 Ml of toluene were used.

EXAMPLE 6

This example illustrated the preparation of 1-methyl-2-propoxyethyl-3,5-dinitrobenzoates according to the procedure described in Example 1 except 42.42 grams (0.2 moles) of 3,5-dinitrobenzoic acid, 24.81 grams (0.21 moles) of propyl propasol, 3.3 grams of Amberlyst 15 (as catalyst) and 25 Ml of toluene were used.

EXAMPLE 7

This example illustrated the preparation of 2-(2-propoxyethoxy) ethyl- 3,5-dinitrobenzoates according to the procedure described in Example 1 except 42.42 grams (0.2 moles) of 3,5-dinitrobenzoic acid, 27.8 grams (0.21 moles) of 2-(2-propoxyethoxy)ethanol, 5 grams of Amberlyst 15 (as catalyst) and 25 Ml of toluene were used.

EXAMPLE 8

This example illustrated the preparation of the 2-propoxyethyl-3,5-dinitrobenzoate according to the procedure described in Example 1 except 42.42 grams (0.2 moles) of 3,5-dinitrobenzoic acid, 18.5 grams (0.21 moles) of 2-propoxyethanol, 5 grams of Amberlyst 15 (instead of methane sulfonic acid) and 25 mL of toluene were used.

Although example 1-8 are specific in examples of the preparation of markers of formula (I) using the general procedure of Example 1, it should be appreciated that this same technique can be applied with equal ease to any other alcohol or analogous thiol and listed and generally enumerated in the specification.

EXAMPLE 9

This example illustrated the general preparation of 3,5-dinitrobenzoates and thio benzoates by esterification of the acid chloride of 3,5-dinitrobenzoic acid (DNBC) with a suitable alcohol or thiol. This particular example depicts the preparation of the 2-butoxyethyl ester of 3,5-dinitrobenzoic acid.

To a glass reaction flask was added 115.3 grams (0.5 moles) of 3,5-dinitrobenzoyl chloride. Then 65 grams (0.55 moles) of 2-butoxyethanol was added all at once and a slow nitrogen purge through the vessel and into a water trap was begun. The mixture was stirred without heating for thirty minutes then warmed to

80 ° C for thirty minutes. Then the product was washed twice with 50 Ml of 1.0 M aqueous potassium carbonate and once with water. The vessel was then heated to 140 ° C and vacuum was applied to remove residual water and alcohol.

EXAMPLE 10

This example illustrated the preparation of the ethyl-3,5-dinitrobenzoates according to Example 9 except that 23.06 grams (0.1 moles) of DNBC and 50 mL of ethanol were used.

EXAMPLE 11

This example illustrated the preparation of the pentyl-3,5-dinitrobenzoates according to Example 9 except that 23.06 grams (0.1 moles) of DNBC and 9.25 grams (1.2 equivalence) of pentanol were used.

EXAMPLE 12

This example illustrated the preparation of the hexyl-3,5-dinitrobenzoates according to Example 9 except that 23.06 grams (0.1 moles) of DNBC and 10.73 grams (1.2 equivalence) of hexanol were used.

EXAMPLE 13

This example illustrated the preparation of the octyl-3,5-dinitrobenzoates according to Example 9 except that 23.06 grams (0.1 moles) of DNBC and 13.76 grams (1.2 equivalence) of octanol were used.

EXAMPLE 14

This example illustrated the preparation of the decyl-3,5-dinitrobenzoates according to Example 9 except that 23.06 grams (0.1 moles) of DNBC and 16.62 grams (1.2 equivalence) of decanol were used.

EXAMPLE 15

This example illustrated the preparation of the 2-(2-butoxyethoxy) ethyl- 3,5-dinitrobenzoates according to Example 9 except that 23.06 grams (0.1 moles) of DNBC and 17.33 grams (1.2 equivalence) of 2-(2-butoxyethoxy)ethanol were used.

EXAMPLE 16

This example illustrated the preparation of the 2-(2-methoxyethoxy) ethyl- 3,5-dinitrobenzoates according to Example 9 except that 23.06 grams (0.1 moles) of DNBC and 12.62 grams (1.2 equivalence) of 2-(2-methoxyethoxy)ethanol were used.

EXAMPLE 17

This example illustrated the preparation of the 2-methoxyethyl-3,5-dinitrobenzoates according to Example 9 except that 23.06 grams (0.1 moles) of DNBC and 8.00 grams (1.2 equivalence) of 2-methoxyethanol were used.

EXAMPLE 18

This example illustrated the preparation of the 2-methyl-1-pentyl-3,5-dinitrobenzoates according to Example 9 except that 15.00 grams (0.065 moles) of DNBC and 8.00 grams (1.2 equivalence) of 2-methyl-1-pentanol were used.

EXAMPLE 19

This example illustrated the preparation of the 4-methyl-2-pentyl-3,5-dinitrobenzoates according to Example 9 except that 15.00 grams (0.065 moles) of DNBC and 8.00 grams (1.2 equivalence) of 4-methyl-2-pentanol were used.

EXAMPLE 20

This example illustrated the preparation of the 2,4-dimethyl-3-pentyl-3,5-dinitrobenzoates according to Example 9 except that 15.00 grams (0.065 moles) of DNBC and 9.07 grams (1.2 equivalence) of 2,4-dimethyl-3-pentanol were used.

EXAMPLE 21

This example illustrated the preparation of the 1-heptyl-3,5-dinitrobenzoates according to Example 9 except that 15.00 grams (0.065 moles) of DNBC and 9.07 grams (1.2 equivalence) of 1-heptanol were used.

EXAMPLE 22

This example illustrated the preparation of the 2-octyl-3,5-dinitrobenzoates according to Example 9 except that 15.00 grams (0.065 moles) of DNBC and 10.17 grams (1.2 equivalence) of 2-octanol were used.

EXAMPLE 23

This example illustrated the preparation of the 3,5,5-trimethyl-1-hexyl 3,5-dinitrobenzoates according to Example 9 except that 15.00 grams (0.065 moles) of DNBC and 11.25 grams (1.2 equivalence) of 3,5,5-trimethyl-1-hexanol were used.

EXAMPLE 24

This example illustrated the preparation of the 3,7-dimethyl-1-octyl-3,5-dinitrobenzoates according to Example 9 except that 15.00 grams (0.065 moles) of DNBC and 12.35 grams (1.2 equivalence) of 3,7-dimethyl-1-octanol were used.

EXAMPLE 25

This example illustrated the preparation of the 2-methyl-1-butyl-3,5-dinitrobenzoate according to Example 9 except 2-methyl-1-butanol was used in place of 2-butoxyethyanol.

EXAMPLE 26

This example illustrated the preparation of the 3-methyl-1-butyl-3,5-dinitrobenzoate according to Example 9 except 3-methyl-1-butanol was used in place of 2-butoxyethyanol.

EXAMPLE 27

This example illustrated the preparation of the methyl-3,5-dinitrobenzoate according to Example 9 except methanol was used in place of 2-butoxyethyanol.

EXAMPLE 28

This example illustrated the preparation of the 2-ethyl-1-butyl-3,5-dinitrobenzoate according to Example 9 except 2-ethyl-1-butanol was used in place of 2-butoxyethyanol.

Although example 9-28 are specific examples of the preparation of markers of formula (I) using the general procedure of Example 9, it should be appreciated that this same technique can be applied with equal ease to any other alcohol or analogous thiol and listed and generally enumerated in the specification.

EXAMPLE 29

This example illustrated the general preparation of 3,5-dinitrobenzamides by amidization of the acid chloride of 3,5-dinitrobenzoic acid (DNBC) with a suitable amine. This particular example depicts the preparation of N,N-dibutyl-3,5-dinitrobenzamide.

To a glass reaction flask was added 46.1 g (0.2 moles) of 3,5-dinitrobenzoyl chloride and 120 grams of toluene. Then 25.9 grams (0.2 moles) of dibutylamine was added over twenty minutes followed by 20.3

grams (0.2 moles) of triethylamine added over ten minutes. The mixture was warmed to 60° C and vigorously stirred for thirty minutes. Then the mixture was washed twice with 100 Ml of water, followed by aqueous potassium carbonate and water again. The vessel was then set up for distillation and heated to 140° C to remove residual water and toluene.

EXAMPLE 30

This example illustrated the preparation of the N,N-dipenyl-3,5-dinitrobenzamide according to Example 29 except that 23.06 grams (0.1 moles) of DNBC and 16.52 grams (1.05 equivalents) of dipentylamine were used.

EXAMPLE 31

This example illustrated the preparation of the N-(2-ethylhexyl)-3,5-dinitrobenzamide according to Example 29 except that 23.06 grams (0.1 moles) of DNBC and 26.50 grams (2.05 equivalents) of 2-ethylhexyl amine were used.

EXAMPLE 32

This example illustrated the preparation of the N,N-di(2-ethylhexyl)-3,5-dinitrobenzamide according to Example 29 except that roughly two equivalents of di-(2-ethylhexyl) amine was used instead of dibutyl amine.

Although example 29-32 are specific examples of the preparation of markers of formula (I) using the general procedure of Example 29, it should be appreciated that this same technique can be applied with equal ease to any other amine listed and generally enumerated in the specification.

PREPARATION OF MARKER LIQUID COMPOSITION

EXAMPLE 33

This example illustrated the general preparation and analysis of a liquid hydrocarbon with a marker of the present invention. In particular, this example depicted the marking of isooctane with three markers of formula (I) and a model compound of formula (I) The example also depicted the analysis of the marked isooctane using a GC with an electron capture detector (ECD).

To a volumetric flask was added 5.00 grams of each of the products from Examples 1 (2-ethylhexyl-3,5-dinitrobenzoate), 9 (2-butoxyethyl-3,5-dinitrobenzoate), 29 (N,N-dibutyl-3,5-dinitrobenzamide), and 1,2-diphenyl ethanedione for comparison. The volume was adjusted to 100 mL with toluene and the contents thoroughly mixed. Then 10 µl of this solution was added by syringe to 100 mL of isooctane. The isooctane solution containing the four electron-capture-detection-sensitive compounds was analyzed by gas chromatography with a polydimethylsiloxane capillary column and ECD. The following data was recorded:

TABLE I

| Markers | Retention Time (min.) | Relative Sensitivity |
|---|---|---|
| 1,2-diphenyl ethanedione | 2.06 | 1.00 |
| 2-ethylhexyl-3,5-dinitrobenzoate | 3.44 | 0.62 |
| 2-butoxyethyl-3,5-dinitrobenzoate | 2.78 | 0.68 |
| N,N-dibutyl-3,5-dinitrobenzamide | 4.31 | 1.21 |

EXAMPLE 34

This example illustrated the use of the marker from Example 29 (N,N-dibutyl-3,5-dinitrobenzamide) to mark commercial gasoline. The marker was added to the performance enhancement package and was easily analyzed according to the procedure in Example 33.

1.0 grams of N,N-dibutyl-3,5-dinitrobenzamide was dissolved in a 99.0 grams of a commercial gasoline additive package containing a polymeric detergent, a synthetic carrier oil and a blend of demulsifying

agents. The resulting package was blended into unadditized premium unleaded gasoline at a dosage of 500 ppm (wt/wt). When analyzed by gas chromatography using the same conditions as in Example 33, N,N-dibutyl-3,5-dinitrobenzamide was detected in the flat baseline region of the GC trace at 4.3 minutes after injection. All other electron capture detector sensitive components of the gasoline had passed through the column within three minutes.

Although this example dealt with commercial gasoline, the marked hydrocarbon liquids, as used in the specification and in the claims, is intended to cover a wide variety and type of organic liquids. Thus, typical liquids that may be marked using the invention are such liquids as gasoline and gasoline blending components, diesel fuels, jet fuels, lubricants, heating oils, residual oils resulting from the refining of petroleum, coal oils and the like.

EXAMPLE 35

This example illustrated the found retention times of a series of 28 of the markers of formula (I) using a polydimethylsiloxane capillary column equipped with a flame ionization detector. Although the detector was different than the electron capture detector, the retention times were unaffected being simply the time required for the marker to exit out of the column and to encounter a detector at the column exit.

To a volumetric flask was added 5.00 grams of each of the following markers (M1-M28) from the following examples:

M1 is N-(2-ethylhexyl)-3,5-dinitrobenzamide from Example 31;
M2 is N,N-di(2-ethylhexyl)-3,5-dinitrobenzamide from Example 32;
M3 is 2-(2-hexoxyethoxy)ethyl-3,5-dinitrobenzoate from Example 3;
M4 is N,N-dipentyl-3,5-dinitrobenzamide from Example 30;
M5 is 2-ethylhexyl-3,5-dinitrobenzoate from Example 1;
M6 is hexyl-3,5-dinitrobenzoate from Example 12;
M7 is pentyl-3,5-dinitrobenzoate from Example 11;
M8 is 2-octyl-3,5-dinitrobenzoate from Example 22;
M9 is N,N-dibutyl-3,5-dinitrobenzamide from Example 29;
M10 is 2-methyl-1-pentyl-3,5-dinitrobenzoate from Example 18;
M11 is 3-methyl-1-butyl-3,5-dinitrobenzoate from Example 26;
M12 is 4-methyl-2-pentyl-3,5-dinitrobenzoate from Example 19;
M13 is 3,5,5-trimethyl-1-hexyl-3,5-dinitrobenzoate from Example 23;
M14 is 2-(2-butoxyethoxy)ethyl-3,5-dinitrobenzoate from Example 15;
M15 is 2-methyl-1-butyl-3,5-dinitrobenzoate from Example 25;
M16 is 2-(2-propoxyethoxy)ethyl-3,5-dinitrobenzoate from Example 7;
M17 is 2-(2-methoxyethoxy)ethyl-3,5-dinitrobenzoate from Example 16;
M18 is decyl-3,5-dinitrobenzoate from Examples 2 or 14;
M19 is methyl-3,5-dinitrobenzoate from Example 27;
M20 is 2-methoxyethyl-3,5-dinitrobenzoate from Example 17;
M21 is 2-ethyl-1-butyl-3,5-dinitrobenzoate from Example 28;
M22 is 2,4-dimethyl-3-pentyl-3,5-dinitrobenzoate from Example 20;
M23 is heptyl-3,5-dinitrobenzoate from Example 21;
M24 is 2-hexoxyethyl-3,5-dinitrobenzoate from Example 4;
M25 is 2-propoxyethyl-3,5-dinitrobenzoate from Example 8;
M26 is 3,7-dimethyl-1-octyl-3,5-dinitrobenzoate from Example 24;
M27 is 2-butoxyethyl-3,5-dinitrobenzoate from Example 9;
M28 is octyl-3,5-dinitrobenzoate from Example 13.

The volume was adjusted to 100 Ml with xylene and the contents thoroughly mixed. Then 0.4 µL of this solution was injected into the GC at a 1 to 6 split ratio. The GC was equipped with a 12 meter by 0.53 millimeter polydimethylsiloxane coated capillary column with 1 micrometer coating. The injector port was 275 ° C and column temperature of 230 ° C. Under these conditions the following data set of retention times were recorded:

TABLE II

| Marker | Retention Time |
|---|---|
| M1 | 11.82 |
| M2 | 24.73 |
| M3 | 16.93 |
| M4 | 11.24 |
| M5 | 6.18 |
| M6 | 4.95 |
| M7 | 3.22 |
| M8 | 6.47 |
| M9 | 6.25 |
| M10 | 4.05 |
| M11 | 3.04 |
| M12 | 4.87 |
| M13 | 7.51 |
| M14 | 9.86 |
| M15 | 3.38 |
| M16 | 7.20 |
| M17 | 5.28 |
| M18 | 11.60 |
| M19 | 1.79 |
| M20 | 2.88 |
| M21 | 3.85 |
| M22 | 3.51 |
| M23 | 5.86 |
| M24 | 8.56 |
| M25 | 3.93 |
| M26 | 9.56 |
| M27 | 4.78 |
| M28 | 6.61 |

Although the markers of the present invention have been described hereinabove in some detail, it should be appreciated that a variety of analogous markers will be readily apparent from the specific formulas encompassed in the specification and claimed in the claims. Other markers and compositions which incorporate modification and analogs to the markers, compositions and methods described hereinabove are equally included within this application.

## Claims

**1.** A composition comprising:

a liquid hydrocarbon and one or more marker compounds, said marker being present in sufficient quantity to facilitate subsequent analytical identification and where said marker is a compound of formula (I):

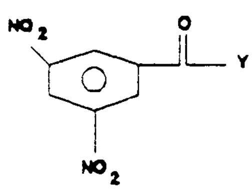

where Y is selected from the group consisting of X-$R^1$ and $NR^2$ $R^3$, where X is either oxygen or sulfur,

where $R^1$ is a organic radical having from about 1 to about 30 carbon atoms, and where $R^2$ and $R^3$ are the same or different organic radical having from about 1 to about 30 carbon atoms.

2. A composition according to claim 1, wherein said marker is a compound of formula (I) where Y is X-$R^1$, where X is oxygen and where $R^1$ is a selected from the group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine.

3. A composition according to claim 2, wherein said marker is a compound of formula (I) where $R^1$ is selected from the group consisting of a linear or branched alkyl group having from about 1 to about 30 carbon atoms, an alkylated glycol and an alkylated polyglycol.

4. A composition according to claim 3, wherein said marker is a compound of formula (I) where $R^1$ is selected from the group consisting of methyl, ethyl, n-propyl, n-butyl, n-pentyl, n-hexyl, n-heptyl, n-octyl, n-nonyl, n-decyl, isopropyl, isobutyl, isopentyl, t-butyl, neopentyl, 2-methyl-1-butyl, 2-ethyl-1-butyl, 2-methyl-1-pentyl, 4-methyl-2-pentyl, 2,4-dimethyl-3-pentyl, 2-ethylhexyl, 3,5,5-trimethyl-1-hexyl, 2-octyl, and 3,7-dimethyl-1-octyl.

5. A composition according to claim 3, wherein said marker is a compound of formula (I) where $R^1$ is selected from the group consisting of 2-(2-methoxyethoxy)ethyl, 2-(2-ethoxyethoxy)ethyl, 2-(2-propoxyethoxy)ethyl, 2-(2-butoxyethoxy)ethyl, (2-pentoxyethoxy)ethyl, 2-(2-hexoxyethoxy)ethyl, 2-methoxyethyl, 2-ethoxyethyl, 2-propoxyethyl, 2-butoxyethyl, 2-pentoxyethyl, 2-hexoxyethyl and 1-methyl-2-propoxyethyl.

6. A composition according to claim 1, wherein said marker is a compound of formula (I) where Y is X-$R^1$, where X is sulfur and where $R^1$ is selected from the group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine.

7. A composition according to claim 6, wherein said marker is a compound of formula (I) where $R^1$ is selected from the group consisting of a linear or branched alkyl group having from about 1 to about 30 carbon atoms, an alkylated glycol and an alkylated polyglycol.

8. A composition according to claim 7, wherein said marker is a compound of formula (I) where $R^1$ is selected from the group consisting of methyl, ethyl, n-propyl, n-butyl, n-pentyl, n-hexyl, n-heptyl, n-octyl, n-nonyl, n-decyl, isopropyl, isobutyl, isopentyl, t-butyl, neopentyl, 2-methyl-1-butyl, 2-ethyl-1-butyl, 2-methyl-1-pentyl, 4-methyl-2-pentyl, 2,4-dimethyl-3-pentyl, 2-ethylhexyl, 3,5,5-trimethyl-1-hexyl, 2-octyl, and 3,7-dimethyl-1-octyl.

9. A composition according to claim 7, wherein said marker is a compound of formula (I) where $R^1$ is selected from the group consisting of 2-(2-methoxyethoxy)ethyl, 2-(2-ethoxyethoxy)ethyl, 2-(2-propoxyethoxy)ethyl, 2-(2-butoxyethoxy)ethyl, (2-pentoxyethoxy)ethyl, 2-(2-hexoxyethoxy)ethyl, 2-methoxyethyl, 2-ethoxyethyl, 2-propoxyethyl, 2-butoxyethyl, 2-pentoxyethyl, 2-hexoxyethyl and 1-methyl-2-propoxyethyl.

10. A composition according to claim 1, wherein said marker is a compound of formula (I) where Y is $NR^2R^3$ and where $R^2$ and $R^3$ are the same or different and selected from the group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine.

11. A composition according to claim 10, wherein said marker is a compound of formula (I) where $R^2$ and

$R^3$ are the same or different and selected from the group consisting a linear or branched alkyl group having from about 1 to about 30 carbon atoms, an alkylated glycol and an alkylated polyglycol.

**12.** A composition according to claim 11, wherein said marker is a compound of formula (I) where $R^2$ and $R^3$ are the same or different and selected from the group consisting of methyl, ethyl, n-propyl, n-butyl, n-pentyl, n-hexyl, n-heptyl, n-octyl, n-nonyl, n-decyl, isopropyl, isobutyl, isopentyl, t-butyl, neopentyl, 2-methyl-1-butyl, 2-ethyl-1-butyl, 2-methyl-1-pentyl, 4-methyl-2-pentyl, 2,4-dimethyl-3-pentyl, 2-ethylhexyl, 3,5,5-trimethyl-1-hexyl, 2-octyl, and 3,7-dimethyl-1-octyl.

**13.** A composition according to claim 1, wherein said marker is present from about 0.5 parts per million to about 25 parts per million.

**14.** A composition according to claim 13, wherein said marker compound is present in an amount from about 1 part per million to about 10 parts per million.

**15.** A composition according to claim 1, wherein said liquid hydrocarbon is selected from the group consisting of crude oil, residual oil, lubricating oil, hydraulic and circulating oils, heating oil, diesel fuel and mid-distillates, jet fuel, aviation fuel, gasoline, gasohol or alcohol fuels, polymer lattices and solutions, MTBE (methyl t-butyl ether), and bulk liquid polymers or mixtures thereof.

**16.** A method for marking a hydrocarbon liquid comprising the steps of adding one or more markers to a liquid hydrocarbon, said marker being present in sufficient quantity to facilitate subsequent analytical identification and quantitation and ranging from about 0.5 ppm to about 50 ppm based on the weight of the liquid hydrocarbon and where said marker is a compound of formula (I)

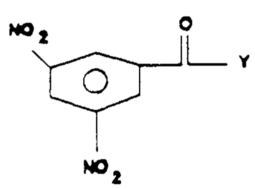

where Y is selected from the group consisting of X-$R^1$ and $NR^2$ $R^3$, where X is either oxygen or sulfur, where $R^1$ is an organic radical having from about 1 to about 30 carbon atoms, and where $R^2$ and $R^3$ are the same or different organic radical having from about 1 to about 30 carbon atoms.

**17.** A method according to claim 16, wherein said marker is a compound of formula (I) where Y is X-$R^1$, where X is oxygen and where $R^1$ is a selected from the group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine.

**18.** A method according to claim 17, wherein said marker is a compound of formula (I) where $R^1$ is selected from the group consisting of a linear or branched alkyl group having from about 1 to about 30 carbon atoms, an alkylated glycol and an alkylated polyglycol.

**19.** A method according to claim 16, wherein said marker is a compound of formula (I) where Y is $NR^2R^3$ and where $R^2$ and $R^3$ are the same or different and selected from the group consisting of a hydrogen atom, a linear or branched alkyl having from about 1 to about 30 carbon atoms, an aryl group having from about 6 to about 30 atoms, a cycloaliphatic group having from about 3 to about 30 carbon atoms, a heterocyclic group, a substituted alkyl group, a substituted aryl group, a diol, a polyol, an alkylated or acylated glycol, an alkylated or acylated polyglycol, and alkylated or acylated ethylene polyamine.

**20.** A method according to claim 19, wherein said marker is a compound of formula (I) where $R^2$ and $R^3$ are

the same or different and selected from the group consisting a linear or branched alkyl group having from about 1 to about 30 carbon atoms, an alkylated glycol and an alkylated polyglycol.

**21.** A method according to claim 16, wherein a set of n markers are added to said liquid at M different quantitative values to form a unique tag for said liquid where n is a number having a value from about 2 to about 15 and M is a number of from about 2 to about 10.

**22.** An analytical method comprising the steps of analyzing a sample of a composition comprising a liquid hydrocarbon and one or more markers by an analytical method sufficient to identify and quantify said markers, said marker being present in sufficient quantity to facilitate subsequent analytical identification and quantitation where said marker is a compound of formula (I):

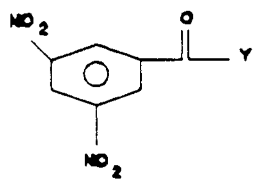

where Y is selected from the group consisting of X-$R^1$ and $NR^2$ $R^3$, where X is either oxygen or sulfur, where $R^1$ is an organic radical having from about 1 to about 30 carbon atoms and where $R^2$ and $R^3$ are the same or different organic radical having from about 1 to about 30 carbon atoms.

**23.** An analytical method according to claim 22 wherein said analytical method is gas chromatography adapted with an electron capture detector.

**24.** An analytical method according to claim 23 wherein said gas chromatogram is equipped with a column including a stationary phase selected from the group consisting of dialkylpolysiloxanes, alkylarylpolysiloxanes, substituted alkylarylpolysiloxanes, copolymers thereof and mixtures thereof and carbowaxes, where the alkyls have from about 1 to about 10 carbon atoms, the aryls have from about 6 to about 14 carbon atoms, and the substituted alkyls are cyanoalkyls, dialkylaminoalkyls, alkoxyalkyls, and haloalkyls.

**25.** An analytical method according to claim 22 wherein said the material is selected from the group consisting of dimethylpolysiloxane, phenylmethyl polysiloxane and cyanopropylphenyl-methyl polysiloxane.

**26.** A method according to claim 22, wherein a set of n markers are analyzed at M different quantitative values to determine a unique tag for said liquid where n is a number having a value from about 2 to about 15 and M is a number of from about 2 to about 10.

**27.** A method for analyzing a liquid composition containing one or more markers comprising the steps of:

1) injecting a liquid composition comprising a liquid hydrocarbon and one or more markers onto a first end of a first GC column through an injector port equipped with a carrier gas supply;

2) separating said markers from a major portion of said liquid into one or more discrete peaks on said first column;

3) opening a valve connected to a second end of said first column for one or more time windows;

4) collecting each of said markers corresponding to each of said windows into a cold trap comprising a first end of a second GC cooled by a coolant;

5) removing said coolant from said cold trap;

6) further separating said makers on said second column; and

7) detecting each of said makers with an electron capture detector equipped with a make up gas,

said first column containing a first column stationary phase and being of a first length sufficient to affect an initial or partial separation of the marker from said major portion of said liquid, said windows corresponding to one or more retention times of said markers and being of sufficient duration to allow

all or nearly all of each marker to pass therethrough, and said second column containing a second column stationary phase being the same or different from said first stationary phase and being of a second length sufficient to separate each of said markers from an other components in said liquid.

European Patent Office

# EUROPEAN SEARCH REPORT

Application Number

EP 92 10 7346

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | US-A-3 366 673 (WAKEMAN ET AL)<br>* the whole document *<br>--- | 1-15 | C10L1/00<br>C10M171/00<br>C10L1/22<br>C10L1/24<br>G01N30/70 |
| A | FR-A-2 563 839 (BANKAMERICA CORP.)<br>* the whole document *<br>--- | 1-15 | |
| A | US-A-3 964 294 (SHAIR ET AL.)<br>* the whole document *<br>--- | 1-27 | |
| A | GB-A-1 111 496 (SHELL)<br>* the whole document *<br>--- | 27 | |
| A | US-A-3 714 812 (DRINKWATER ET AL.)<br>* the whole document *<br>--- | 27 | |
| D,A | US-A-4 141 692 (KELLER)<br>* the whole document *<br>--- | 27 | |
| A | TRAC, TRENDS IN ANALYTICAL CHEMISTRY.<br>vol. 4, no. 2, February 1985, AMSTERDAM-NL<br>pages 55 - 59;<br>BRINKMAN ET AL.: 'LIQUID CHROMATOGRAPHY WITH ON-LINE ELECTRON-CAPTURE DETECTION--A VIABLE WAY TO GO?'<br>* the whole document *<br>--- | 27 | TECHNICAL FIELDS SEARCHED (Int. Cl.5 )<br>C10L<br>C10M<br>G01N |
| A | JOURNAL OF CHROMATOGRAPHY.<br>vol. 92, no. 2, 22 May 1974, AMSTERDAM NL<br>pages 299 - 301;<br>PELLIZZARI: 'HIGH-RESOLUTION ELECTRON CAPTURE GAS-LIQUID CHROMATOGRAPHY'<br>* the whole document *<br>----- | 27 | |

The present search report has been drawn up for all claims

| Place of search | Date of completion of the search | Examiner |
|---|---|---|
| THE HAGUE | 10 JULY 1992 | DE LA MORINERIE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document